# EUROPEAN PATENT APPLICATION

(11) **EP 1 304 109 A1**
(43) Date of publication of application: **23.04.2003**
(21) Application number: 02257190.5
(22) Date of filing: 16.10.2002
(51) Int. Cl.: A61K 31/445

(54) **Liquid for oral administration comprising paroxetine**

(30) Priority: 19.10.2001 CA 2361882
(71) Applicant: Sherman, Bernard Charles, Toronto, Ontario M2L 2K1 (CA)
(72) Inventor: Sherman, Bernard Charles, Toronto, Ontario M2L 2K1 (CA)
(74) Representative: Howard, Paul Nicholas

(57) **Abstract**

An oral liquid pharmaceutical composition comprising paroxetine or a salt thereof and a basic compound which imparts a pH of above 7 to the composition.

## Description

### BACKGROUND OF THE INVENTION

Paroxetine is a compound useful as an antidepressant and is disclosed in U.S. Patent No. 4007196.

Compositions comprising paroxetine as the hydrochloride salt are sold in the United States and elsewhere under the brandname Paxil™. Paxil™ is available as tablets for oral administration, and also as a suspension for oral administration in strengths of 10 mg per 5 mL; i.e. each 5 mL contains paroxetine hcl 11.1 mg, which is equivalent to paroxetine 10 mg.

It is difficult to formulate a suitable oral liquid comprising paroxetine or a paroxetine salt, because paroxetine has a very bitter taste.

US patent no. 5811436 teaches that the bitter taste can be overcome by complexing paroxetine hydrochloride with AMBERLITE IRP-88 resin. The oral liquid (i.e. suspension) is prepared in a conventional manner by mixing paroxetine hydrochloride and AMBERLITE IRP-88 together in an aqueous medium, along with acceptable excipients such as thickeners, humectants, sweeteners, buffering agents, preservatives, colours and flavours.

It is further taught that the amounts of buffering agents are preferably controlled to give a pH of 4 to 6.

While the teaching of US Patent No. 5811436 results in an acceptable product, it is desirable to have an alternate means of making satisfactory paroxetine oral liquids which do not require complexing with AMBERLITE IRP-88.

The object of the present invention is to enable such an oral liquid.

### BRIEF SUMMARY OF THE INVENTION

It has surprisingly been found that the bitterness of an oral liquid comprising paroxetine or a salt thereof can be overcome by adding a basic compound to raise the pH to above 7 and preferably to between 8 and 10.

### DETAILED DESCRIPTION OF THE INVENTION

Compositions according to the present invention are liquids for oral administration comprising paroxetine or a salt thereof, preferably paroxetine hydrochloride, which may be either anhydrous or hemihydrate. The concentration of paroxetine in the liquid will preferably be about 10 mg/5 mL.

The compositions will further comprise a basic compound, such as for example sodium hydroxide, or a sodium salt of a weak acid, such as sodium bicarbonate, sodium carbonate, sodium citrate, disodium phosphate or trisodium phosphate, for the purpose of raising the pH. The basic compound will most preferably be trisodium phosphate. The pH of the composition will be above 7, will more preferably be above 8, will even more preferably be between 8 and 10.

The compositions are prepared in a conventional manner by mixing the paroxetine or salt thereof and basic compound in an aqueous medium.

Other pharmaceutically acceptable excipients may also be added, such as thickeners (in particular Avicel CL611), a humectant such as glycerol, sweeteners such as sorbitol and sodium saccharin, preservatives such as methyl and propyl parabens (which will preferably be dissolved in propylene glycol and then added as a solution in propylene glycol), artificial colours such as FD & C Yellow No. 6, flavours, and an antifoaming agent such as silicone antifoam.

The following example illustrates the present invention:

### EXAMPLE 1

| | Per 10 mL |
|---|---|
| Paroxetine hydrochloride anhydrous | 22.2 mg |
| Trisodium Phosphate Anhydrous | 16.0 mg |
| Avicel CL611 | 300.0 mg |
| Glycerol | 500.0 mg |
| Sorbitol Solution 70% | 4000.0 mg |
| Propylene Glycol | 500.0 mg |
| Methyl Parahydroxybenzoate | 20.0 mg |
| Propyl Parahydroxybenzoate | 6.0 mg |
| Sodium Saccharin | 5.0 mg |
| FD & C Yellow No. 6 Aluminum Lake 40% | 7.0 mg |
| Orange Flavour | 3.0 mg |
| Silicone Antifoam 1510 | 20.0 mg |
| Water | q.s. to 10 mL |

The pH of the liquid of this example is about 8.8.

## Claims

1. An oral liquid pharmaceutical composition comprising paroxetine or a salt thereof, water, and a basic compound which imparts pH of above 7 to the composition.

2. A composition of claim 1 comprising paroxetine hydrochloride.

3. A composition of claim 2 wherein the paroxetine hydrochloride is in the form of the hemihydrate.

4. A system of claim 2 wherein the paroxetine hydrochloride is anhydrous.

5. A composition of any of claims 1 to 4 having pH above 8.

6. A composition of any of claims 1 to 4 having pH between 8 and 10.

7. A composition of any of claims 1 to 6 wherein the basic compound is sodium hydrochloride or a sodium salt of a weak acid.

8. A composition of any of claims 1 to 6 wherein the basic compound is sodium bicarbonate.

9. A composition of any of claims 1 to 6 wherein the basic compound is sodium carbonate.

10. A composition of any of claims 1 to 6 wherein the basic compound is sodium citrate.

11. A composition of any of claims 1 to 6 wherein the basic compound is disodium phosphate.

12. A composition of any of claims 1 to 6 wherein the basic compound is trisodium phosphate.
